# EUROPÄISCHE PATENTSCHRIFT

(11) **EP 3 694 851 B1**
(45) Veröffentlichungstag und Bekanntmachung des Hinweises auf die Patenterteilung: **27.12.2023**
(21) Anmeldenummer: 18786722.1
(22) Anmeldetag: 10.10.2018
(51) Int. Cl.: C07D 405/06, A61K 31/47, A61P 31/04

(54) **IN DER UMWELT ABBAUBARE CHINOLON-ANTIBIOTIKA MIT HEMIAMINAL-STRUKTUREINHEIT**
ENVIRONMENTALLY DEGRADABLE QUINOLONE ANTIBIOTICS HAVING A HEMIAMINAL STRUCTURAL UNIT
ANTIBIOTIQUES DE LA FAMILLE DES QUINOLONES, DÉGRADABLES DANS L'ENVIRONNEMENT, COMPRENANT UN MOTIF STRUCTURAL HÉMIAMINAL

(30) Priorität: 11.10.2017 DE 102017218119
(43) Veröffentlichungstag der Anmeldung: 19.08.2020
(73) Patentinhaber: Leuphana Universität Lüneburg Stiftung Öffentlichen Rechts, 21335 Lüneburg (DE)
(72) Erfinder: KUEMMERER, Klaus, 21337 Lueneburg (DE); LEDER, Christoph, 21403 Wendisch Evern (DE); RASTOGI, Tushar, 89075 Ulm (DE); SUK, Morten, 21337 Lueneburg (DE); PEIFER, Christian, 24354 Soenderby (DE)
(74) Vertreter: Wichmann, Hendrik
(86) Internationale Anmeldenummer: PCT/EP2018/077582
(87) Internationale Veröffentlichungsnummer: WO 2019/072905

(56) Entgegenhaltungen:
- EP-A1- 0 700 912
- JP-A- S61 152 682
- US-A1- 2017 189 556
- KLAUS KÜMMERER: "Sustainable from the very beginning: rational design of molecules by life cycle engineering as an important approach for green pharmacy and green chemistry", GREEN CHEMISTRY, Bd. 9, Nr. 8, 1. Januar 2007 (2007-01-01), Seiten 899-907, XP055524225, GB ISSN: 1463-9262, DOI: 10.1039/b618298b
- T TAKENOUCHI ET AL: "Hydrophilicity of quinolones is not an exclusive factor for decreased activity in efflux-mediated resistant mutants of Staphylococcus aureus.", ANTIMICROBIAL AGENTS AND CHEMOTHERAPY, Bd. 40, Nr. 8, 1. August 1996 (1996-08-01) , Seiten 1835-1842, XP055524122, US ISSN: 0066-4804, DOI: 10.1128/AAC.40.8.1835
- ANGELES DE LA CRUZ ET AL: "Synthesis and biological evaluation of 4-quinolone ribosides", JOURNAL OF THE CHEMICAL SOCIETY, PERKIN TRANSACTIONS 1, Nr. 7, 1. Januar 1993 (1993-01-01), Seite 845, XP055524195, ISSN: 0300-922X, DOI: 10.1039/p19930000845
- FABYANA A. SOARES ET AL: "Molecular design, synthesis and biological evaluation of 1,4-dihydro-4-oxoquinoline ribonucleosides as TcGAPDH inhibitors with trypanocidal activity", BIOORGANIC & MEDICINAL CHEMISTRY LETTERS, Bd. 23, Nr. 16, 1. August 2013 (2013-08-01), Seiten 4597-4601, XP055524335, AMSTERDAM, NL ISSN: 0960-894X, DOI: 10.1016/j.bmcl.2013.06.029
- MAGDALENA NASCIMENTO RENNÓ ET AL: "Kinetics and docking studies of two potential new inhibitors of the nucleoside hydrolase from Leishmania donovani", EUROPEAN JOURNAL OF MEDICINAL CHEMISTRY, Bd. 56, 1. Oktober 2012 (2012-10-01), Seiten 301-307, XP055524345, FR ISSN: 0223-5234, DOI: 10.1016/j.ejmech.2012.07.052
- Carla Verônica ET AL: "Synthesis and Anti-HSV-1 Activity of 1,4-dihydro-4-oxoquinoline Ribonucleosides", Letters in Drug Design & Discovery, 1. Januar 2007 (2007-01-01), Seiten 404-409, XP055524347, Gefunden im Internet: URL:https://www.google.com/url?sa=t&rct=j& q=&esrc=s&source=web&cd=1&ved=2ahUKEwjAvf- zgNneAhUIU1AKHSekC0kQFjAAegQIBBAB&url=http s%3A%2F%2Fwww.ingentaconnect.com%2Fcontent %2Fben%2Flddd%2F2007%2F00000004%2F00000006 %2F004aj%3Fcrawler%3Dtrue&usg=AOvVaw0UQtMG enbkq21K0O0pu1_4
- ED T. BUURMAN ET AL: "Antimicrobial Activity of Adenine-Based Inhibitors of NAD + -Dependent DNA Ligase", ACS MEDICINAL CHEMISTRY LETTERS, Bd. 3, Nr. 8, 16. Juli 2012 (2012-07-16), Seiten 663-667, XP055524468, ISSN: 1948-5875, DOI: 10.1021/ml300169x
- JIA XUE-DONG ET AL: "Synthesis andin vitroantitumor activity of novel naphthyridinone derivatives", CHINESE CHEMICAL LETTERS, ELSEVIER, AMSTERDAM, NL, vol. 28, no. 2, 25 July 2016 (2016-07-25), pages 235-239, XP029893527, ISSN: 1001-8417, DOI: 10.1016/J.CCLET.2016.07.024
- TOMITA KYOJI ET AL: "Synthesis and Structure-Activity Relationships of Novel 7-Substituted 1,4-Dihydro-4-oxo-1-(2-thiazolyl)-1,8-naph thyridine-3-carboxylic Acids as antitumor Agents", JOURNAL OF MEDICINAL CHEMISTRY, AMERICAN CHEMICAL SOCIETY, US, vol. 45, no. 25, 1 January 2002 (2002-01-01), pages 5564-5575, XP002450261, ISSN: 0022-2623, DOI: 10.1021/JM010057B
- DATABASE CAPLUS [Online] CHEMICAL ABSTRACTS SERVICE, COLUMBUS, OHIO, US; 1992, Chen, J. X.; Guo, H. Y.: "Pyridonecarboxylic acids as antibacterial agents. X. Synthesis and structure-activity relationships of 1,7-disubstituted-6-fluoro-1,4-dihydro-4-o xoquinoline-3-carboxylic acids", XP55917490, Database accession no. 1992:128614
- DATABASE CAPLUS [Online] 1 January 1991 (1991-01-01), Chen ET AL: "Pyridonecarboxylic acids as antibacterial agents. X. Synthesis and structure-activity relationships of 1,7-disubstituted-6-fluoro-1,4-dihydro-4-o xoquinoline-3-carboxylic acids", XP055917490, Database accession no. 1992:128614
- DATABASE REGISTRY [Online] 1 January 2008 (2008-01-01), Registry: "3-Quinolinecarboxylic acid", XP055917504, Database accession no. 1089336-20-8, 1089336-14-0

## Beschreibung

Die vorliegende Erfindung betrifft neue Chinolon-Antibiotika mit Hemiaminal-Struktureinheit, wie in den Ansprüchen definiert, und diese Chinolon-Antibiotika zur medizinischen Verwendung, wie in den Ansprüchen definiert.

### Stand der Technik

Da sich die instrumentelle Analytik in den letzten Jahrzehnten rasant weiterentwickelt hat, wurden bisher schon annähernd zweihundert verschiedene Pharmazeutika weltweit im Ablauf von Kläranlagen, Oberflächengewässern, Grundwasser und vereinzelt auch Trinkwasser im Bereich von ng/L bis µg/L detektiert. Diese Mikroverunreinigungen werden daher mittlerweile als Risiko für die Wasserqualität und ein nachhaltiges Management der Wasserressourcen wahrgenommen. In dem Zusammenhang ist auch sehr wenig über das Auftreten und das umweltwissenschaftliche Schicksal der Zerfalls- und Abbauprodukte der applizierten oder unsachgemäß entsorgten Pharmazeutika bekannt. Persistenz, Akkumulation, Metabolisierung durch Mikroorganismen, Tiere und Pflanzen, Reaktion mit Sauerstoff oder Beeinflussung durch Licht können die Ausgangsstoffe so verändern, dass sich die Toxizität der Substanzen verändern kann und damit die Trinkwasserressourcen beeinträchtigt werden können.

Besonders problematisch sind Antibiotika. Antibiotika werden zur Behandlung von Infektionen mit pathogenen Bakterien genutzt. Nutzung von nicht abbaubaren Antibiotika durch menschliche Patienten oder bei Nutztieren kann zur Akkumulation im Wasser führen und könnte anschließend die Resistenzentwicklung fördern, wenn große Areale mit relevanten Bakterien ausreichende Wirkstoffkonzentrationen von Antibiotika haben und somit ständigen Selektionsdruck auf die Bakterien ausüben.

Zu nennen ist z.B. das bekannte Antibiotikum Ciprofloxacin. Es gehört zur zweiten Generation der Gruppe der Fluorochinolone und ist ein Breitband-Antibiotikum, das die prokaryonten-spezifische DNA-Gyrase inhibiert. Das Schicksal von Ciprofloxacin in der Umwelt ist mittlerweile in den Blickpunkt geraten, weil die Substanz im Abwasser, insbesondere von Krankenhäusern, und in Kläranlagen wie auch im Klärschlamm detektiert wurde. Im Abwasser von Schweizer Krankenhäusern wurden Konzentrationen zwischen 2- 83 µg/l gemessen. In Kläranlagen wird es lediglich teilweise durch Adsorption an den Klärschlamm entfernt. Es wird nicht von Mikroorganismen in Kläranlagen abgebaut (Kümmerer et al. 2000). Daher kann nicht ausgeschlossen werden, dass biologische Abbauprozesse in Kläranlagen mit hoher Antibiotika-Aufnahme aufgrund der Akkumulation der antibiotischen Aktivität empfindlich gestört werden und Resistenzbildungen oder - selektionen gefördert werden.

Es ist bekannt, dass Hemiaminale insbesondere bei niedrigem pH-Werten instabil sind. Ob dies auf derivatisierte Fluorchinolone ebenfalls zutrifft, ist unbekannt.

Takenouchi et al. offenbaren verschiedene Fluorchinolon-Derivate (z.B. Ciprofloxacin-Derivate), um deren Löslichkeiten und Aktivitäten zu analysieren (Antimicrobial Agents and Chemotherapy, 1996, 40(8), 1835-1842). Unter den gezeigten Ciprofloxacin-Derivaten gibt es auch ein Hemiaminal-Rest in der Form -C(OR)(NR2)- (Substanz 33, in der Publikation als R1 bezeichnet). Jedoch wird hier lediglich eine ähnliche Struktur offengelegt ohne Bezug auf eine verbesserte Abbaubarkeit. Die Verbindung 33 von Takenouchi weist allerdings keinen ausreichenden +I-Effekt auf, sodass dass die antibiotische Aktivität zu niedrig ist.

Weiterhin offenbaren Radi et al. verschiedene Fluorchinolon-Derivate (z.B. Ofloxacin-Derivate) um weitere antibakteriell wirkende Substanzen zu synthetisieren ("Preparation of some 2,3-dihydro-7-oxo-7H-pyrido[1,2,3-de][1,4]benzoxazine derivatives." Collection of Czechoslovak Chemical Communications, 1992, 57(1), 216-18). Unter den gezeigten Ofloxacin-Derivaten gibt es auch einen Hemiaminal-Rest in der Form -C(OH)(NR2)-. Die Patentschrift EP0373531 offenbart ebenfalls Ofloxacin-Derivate mit einem Hemiaminal-Rest in der cyclischen Ether-Form -C(OR)(NR2)-, mit dem Ziel neue und wirkungsvolle Antibiotika bereitzustellen, nicht aber um eine verbesserte Abbaubarkeit zu erreichen.

WO2015/189560 offenbart Quinolone-Derivate mit gängigen Substituenten. Hemiaminalether werden jedoch nicht offenbart. Gemäß Anspruch 1 des Dokuments kann R2 einen heterozyklischen Ring mit Z bilden. Dies entspricht allerdings nicht den vorliegend beanspruchten Strukturen. Ähnliche Verbindungen sind auch in DE 692 32 134 bzw. WO 01/36408 A1 beschrieben. DE 692 32 134 beschreibt ebenfalls keine Hemiaminalether.

DE 36 32 222 A1 offenbart antibakterielle Wirkstoffe, wobei gemäß Anspruch 1 der Substituent R¹ 2-Hydroxyethyl oder Methoxy sein kann. Beide Möglichkeiten entsprechen allerdings nicht den Vorgaben des N-Substituenten der vorliegenden Patentansprüche. Hemiaminalether werden auch generell nicht offenbart.

Im Rahmen der vorliegenden Erfindung wurde eines dieser Derivate (Marbofloxacin) bei niedrigem pH getestet und es wurde kaum Instabilität beobachtet. Diese Art von Hemiaminal weist daher keine besondere Abbaubarkeit in der Umwelt auf. Da bislang die generelle Stabilität von Antibiotika im Pharma-Bereich im Vordergrund stand, wurden keine abbaubaren Wirkstoffe entwickelt. Im Gegensatz dazu zielt die vorliegende Erfindung auf eine Balance zwischen Abbaubarkeit in der Umwelt und ausreichender Stabilität und Wirksamkeit in der Therapie ab.

Weitere Chinolon-Derivate sind in den folgenden Veröffentlichungen beschrieben:
- Klaus Kümmerer: "Sustainable from the very beginning: rational design of molecules by life cycle engineering as an important approach for green pharmacy and green chemistry", GREEN CHEMISTRY, Bd. 9, Nr. 8, 2007, Seiten 899-907
- EP0700912
- ANGELES DE LA CRUZ ET AL: "Synthesis and biological evaluation of 4-quinolone ribosides", JOURNAL OF THE CHEMICAL SOCIETY, PERKIN TRANSACTIONS 1, Nr. 7, 1993, Seite 845
- FABYANA A. SOARES ET AL: "Molecular design, synthesis and biological evaluation of 1,4-dihydro-4-oxoquinoline ribonucleosides as TcGAPDH inhibitors with trypanocidal activity", BIOORGANIC &MEDICINAL CHEMISTRY LETTERS, Bd. 23, Nr. 16, 2013, Seiten 4597-4601
- Magdalena Nascimento Renno ET AL: "Kinetics and docking studies of two potential new inhibitors of the nucleoside hydrolase from Leishmania donovani", European Journal of Medicinal Chemistry, Bd. 56, 2012, Seiten 301-307
- Carla Veronica ET AL: "Synthesis and Anti-HSV-1 Activity of 1,4- dihydro-4-oxoquinoline Ribonucleosides", Letters in Drug Design & Discovery, 2007, Seiten 404-409
- ED T. BUURMAN ET AL: "Antimicrobial Activity of Adenine-Based Inhibitors of NAD + - Dependent DNA Ligase", ACS MEDICINAL CHEMISTRY LETTERS, Bd. 3, Nr. 8, 2012, Seiten 663-667
- US 2017/189556
- JP S61 152682 A
- TOMITA KYOJI ET AL: "Synthesis and Structure-Activity Relationships of Novel 7-Substituted 1,4-Dihydro-4-oxo-1-(2-thiazolyl)-1,8-naphthyridine-3- carboxylic Acids as antitumor Agents", JOURNAL OF MEDICINAL CHEMISTRY, Bd. 45, Nr. 25, 2002, Seiten 5564-5575, XP002450261,
- JIA XUE-DONG ET AL: "Synthesis and in vitro antitumor activity of novel naphthyridinone derivatives", CHINESE CHEMICAL LETTERS, ELSEVIER, Bd. 28, Nr. 2, 25. Juli 2016 (2016-07-25), Seiten 235-239, XP029893527,
- DATABASE CAPLUS [Online] 1. Januar 1991, Chen ET AL: "Pyridonecarboxylic acids as antibacterial agents. X. Synthesis and structure-activity relationships of 1,7-disubstituted-6- fluoro-1,4-dihydro-4-oxoquinoline-3-carboxylic acids", XP055917490, Database accession no. 1992:128614
- DATABASE REGISTRY [Online] 1. Januar 2008, Registry: "3-Quinolinecarboxylic acid", XP055917504, Database accession no. 1089336-20-8, 1089336-14-0.

### Zusammenfassung der Erfindung

Der Vorteil der hierin offenbarten Verbindungen gegenüber bekannten antimikrobiellen Wirkstoffen, die auf Stabilität optimiert sind, liegt in der chemischen oder biologischen Abbaubarkeit in der Umwelt. Neben den toxischen Umweltaspekten wirkt diese Abbaubarkeit auch dem Selektionsdruck auf die Umweltbakterien und die damit einhergehende Resistenzausbildung oder -selektion entgegen. Insbesondere ist die aktive Wirkung zeitlich auf die eigentliche humane oder veterinäre Therapie begrenzt, und der nachfolgende Abbau in der Umwelt sorgt für eine gezielte Inaktivierung. Damit könnte der auf Umweltkeime einwirkende Selektionsdruck und die Resistenzausbildung weiter wirksam eingedämmt werden.

Im Rahmen der vorliegenden Erfindung wurden durch gezielte molekulare Strukturveränderungen umweltfreundlichere Antibiotikavarianten auf Basis der Fluorchinolon-Grundstruktur entwickelt, um nachfolgend die durch Pharmazeutika verursachten Gefahren auch für die Wasserqualität abzumildern, indem die Abbaubarkeit verbessert wird oder zumindest die Aktivität und die Toxizität von nicht-abbaubaren Metaboliten, Transformationsprodukten und Fragmenten neutralisiert wird. Die hierin beschriebenen Verbindungen können als "Hemiaminale" oder genauer "O- derivatisierte Hemiaminale" bezeichnet werden, wobei der Name "Hemiaminal" bedeutet, dass eine der beiden verätherten Sauerstoffatome eines Acetals durch ein N-Atom (Äther durch Aminogruppe) ersetzt ist. Entscheidend für die erfindungsgemäße Abbaubarkeit ist das Struktur-Element C-O-C-N am Chinolon N1. Die Hydrolyse findet hierbei an der N-C-Bindung statt, wobei die Hydrolyse des Halbaminals zum Alkohol bzw. Halbacetal führt, das dann weiter hydrolysiert wird zum Aldehyd, da das N- Atom im Ring als Teil des Grundgerüstetes verbleibt (s. auch Struktur Cip_d_CP).

Somit führt die Berücksichtigung von Umweltaspekten zu einer wechselseitigen Verbesserung der Medikamente. Dadurch können neue Medikamente entstehen, die pharmakologisch und umweltwissenschaftlich verbessert sind.

Die vorliegend beschriebenen Verbindungen werden durch Verknüpfung der Chinolon-Grundstruktur mit chemisch/abiotisch abbaubaren Linkern hergestellt.

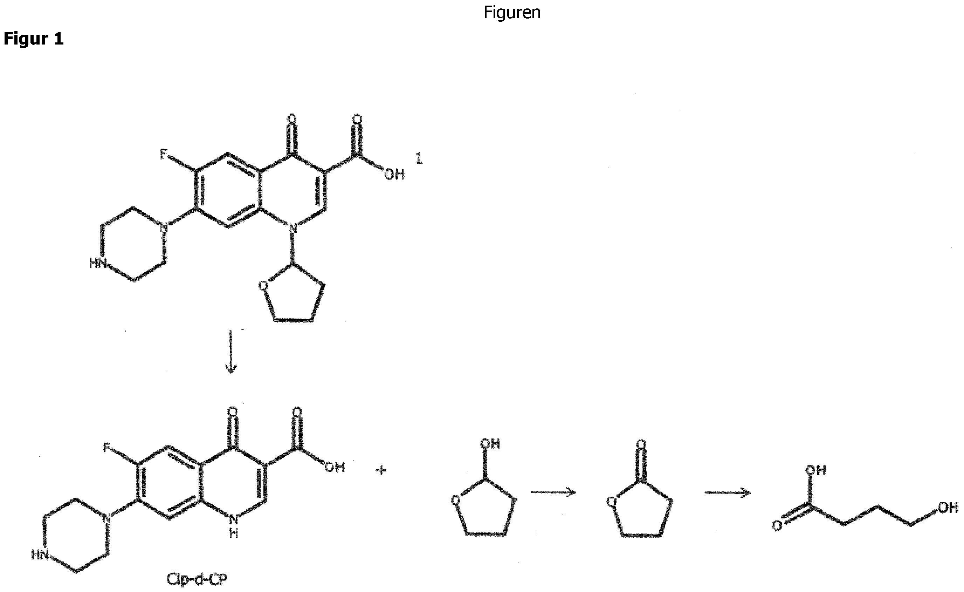

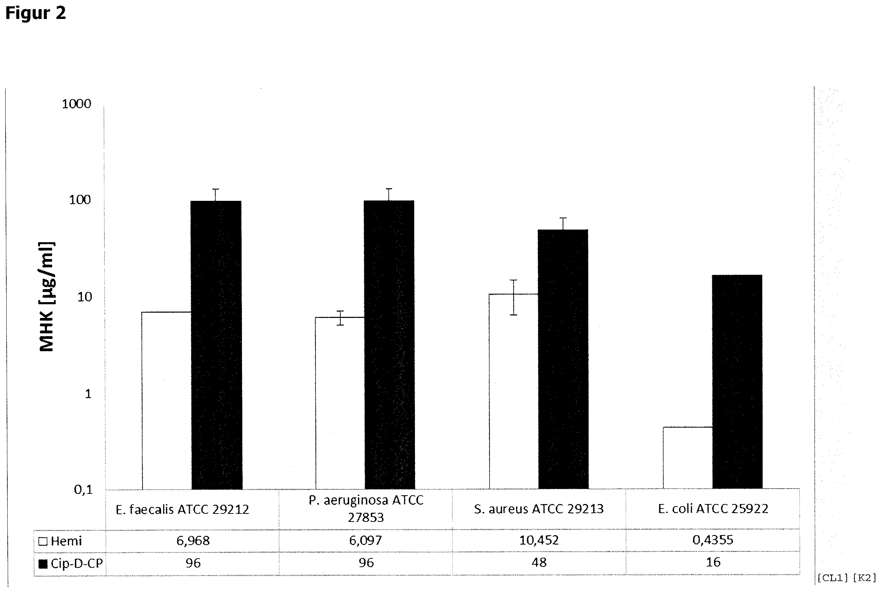

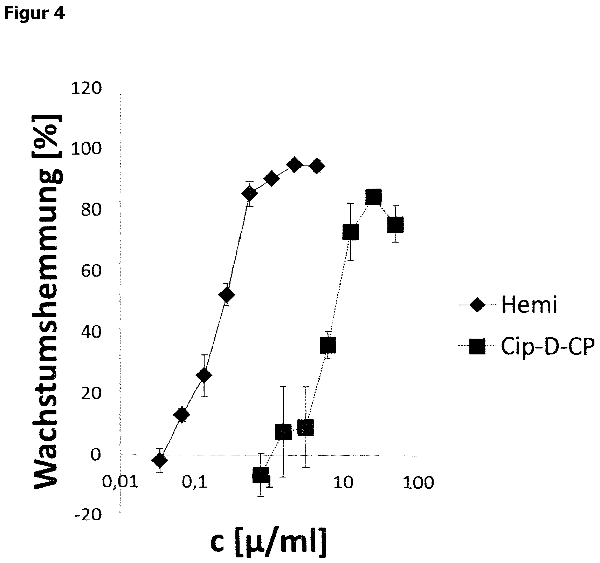

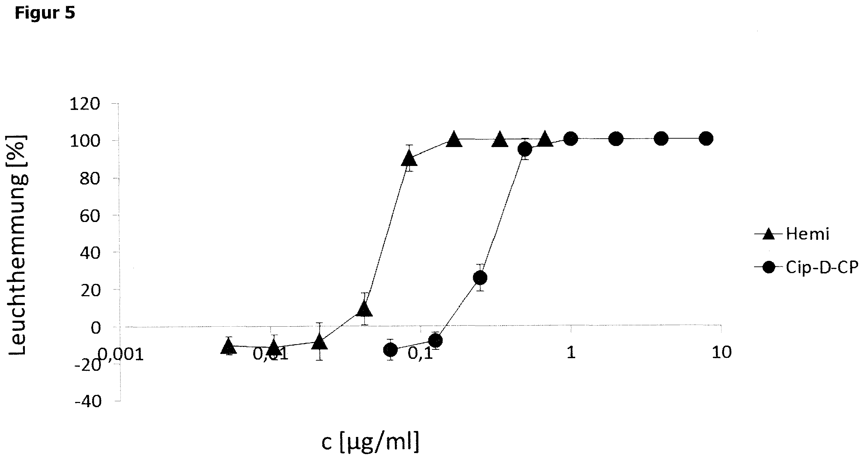

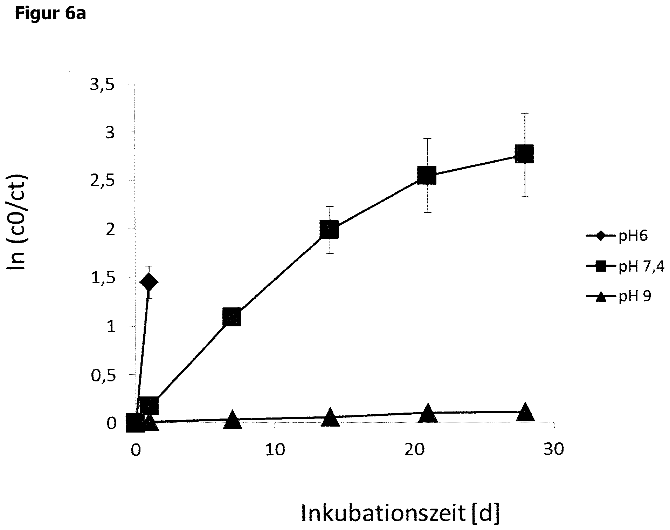

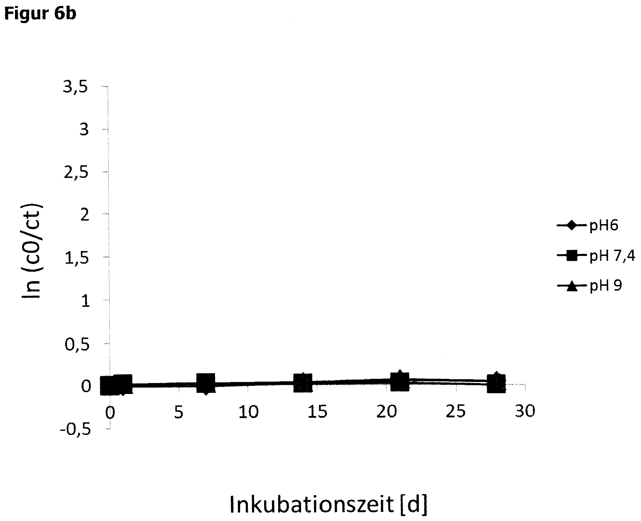

Verbindung 1 (siehe Figur 1) wurde hinsichtlich seiner Aktivität getestet (siehe Figuren 2-4) und mit dem verbleibenden Fragment Cip-d-CP (Ciprofloxacin delta Cycloproyl, d.h. das Cipro-Grundgerüst ohne Cyclopropyl an N1) verglichen. Die Aktivität von Verbindung 1 liegt in *E. coli* (ATCC 23716) im Bereich der mittelstarken Fluorchinolone (MIC = ca. 0,1-1 µg/ml) (siehe Figur 4). In anderen pathogenen Bakterien liegt sie über den Chinolonen der ersten Generation, aber unterhalb etablierter Fluorchinolone der zweiten Generation. Weiterhin konnte gezeigt werden, dass der labile Linker unter physiologischen Bedingungen langsam und bei niedrigem pH schnell abgespalten wird (Figuren 6a und 6b). Das verbleibende Fragment nach Abspaltung des Linkers (Cip-d-CP) war deutlich schwächer aktiv als die Ausgangssubstanz (Figur 2). Auch unter den Testbedingungen des standardisierten Abbautests "geschlossener Flaschentest" (OECD 301D), der näherungsweise Verhältnisse in Oberflächengewässern simuliert, findet der Zerfall von Verbindung 1 in Cip-d-CP statt. Weder das verbleibende Fragment noch die Ausgangssubstanz Ciprofloxacin waren unter den gewählten pH-Bedingungen instabil. Auch in den Umweltbakterien *V. fischeri* zeigte sich eine deutliche Abschwächung der antibiotischen Aktivität nach Abspaltung des Linkers (Figur 5).

Die Instabilität bei niedrigen pH-Werten macht eine enterische Formulierung oder intravenöse Applikation des Arzneimittels besonders interessant, um eine Freisetzung im sauren Magenmilieu zu verhindern.

Das Risiko, dass sich gegen diese neuen Antibiotika-Varianten Resistenzen bilden, sollte dadurch verringert sein, dass es nach Ausscheidung zu einer raschen Inaktivierung kommt und somit über die eigentliche Behandlung hinaus kein Selektionsdruck, z.B. auf Bakterien im Abwasser und in der Umwelt, ausgeübt wird. Dadurch wird das Problem gelöst, dass die starke Anwendung von Antibiotika dazu führt, dass die nicht-abbaubaren Antibiotika (z.B. Fluorchinolon-Antibiotika) noch im Abwasser, in der Kläranlage und in der Umwelt aktiv bleiben und daher die Bildung oder Selektion von Resistenzen gefördert würde.

Da die Inaktivierung unter bestimmten pH-Bedingungen abiotisch abläuft, könnte es weniger wahrscheinlich sein, dass diese Inaktivierung von Bakterien durch Mutation von Enzymen beschleunigt wird. D.h. die vorliegend beschriebenen Verbindungen führen wahrscheinlich nicht zu einer höheren intrinsischen Anfälligkeit für Resistenzbildungen.

Für nicht-zyklische, kurze Hemiaminale wird aufgrund der Struktur-Aktivitäts-Eigenschaften an dieser Position eine erhöhte antibakterielle Aktivität erwartet.

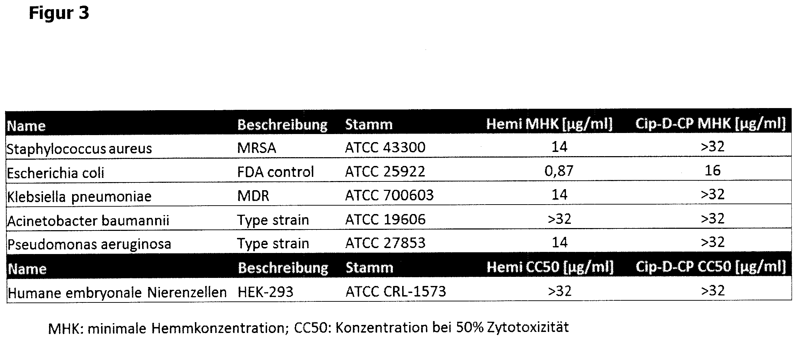

Es konnte bisher gezeigt werden, dass Verbindung 1 nicht durch intestinale und hepatische Enzyme abgebaut wird. Dies deutet darauf hin, dass das Molekül als Therapeutikum nutzbar sein wird. Des Weiteren konnten bisher keine Hinweise auf einen Zytotoxizität in humanen Zellen (HEK 293) durch Verbindung 1 festgestellt werden (Figur 3, HEK-293).

Die vorliegende Erfindung beschreibt neue antibiotische Substanzen auf Grundlage des bekannten Fluorchinolons Ciprofloxacin. Diese neuen Substanzen sollen in der Umwelt abgebaut werden können (umweltfreundliche Ciprofloxacin-Derivate). Heutzutage sind viele Medikamente sowohl im Abwasser als auch im Trinkwasser nachweisbar. Eine Verringerung bzw. ein Abbau dieser Wirkstoffe würde neben den toxischen Umweltaspekten auch den Selektionsdruck auf die Umweltbakterien und die damit einhergehende Resistenzausbildung entgegenwirken.

Ausgehend von Ciprofloxacin als Breitbandantibiotikum der 2. Generation der Fluorchinolone modifizierten die Erfinder die Fluorchinolon-Grundstruktur, um mittels eines eingebrachten pH-labilen Hemiaminal-Restes ein biologisch (enzymatisch) oder abiotisch (pH-abhängig) spaltbares Antibiotikum zu erhalten. Halbaminale haben die allgemeine Struktur -C(OH)(NR2)-. Verbindung 1 enthält einen cyclischen Ether-Rest (-C(OR)(NR2)-).

Dieses Derivat (Verbindung 1) wurde im Labor beispielsweise gegen Stämme von *E. coli, K. pneumoniae, P. aeruginosa* sowie *V. fischeri* getestet und zeigte zu mittelstarken Fluorchinolonen eine vergleichbare antibakterielle Aktivität. Labortests zeigten, dass unter physiologischen Bedingungen (pH= 7,4) dieser Rest innerhalb von 7 Tagen nur langsam abgebaut wurde, während es bei einem niedrigen pH von vier (z.B. im Urin) innerhalb dieser Zeitspanne zum größten Teil abgebaut wird. Das verbleibende Fragment nach Abspaltung war dabei deutlich schwächer aktiv (siehe auch Figuren 2 und 3).

Ausgehend von diesen Ergebnissen wird angenommen, dass man Chinolon-Antibiotika mit pH-labilen Hemiaminal-Resten derivatisieren kann, damit diese in der Umwelt besser abgebaut werden und gleichzeitig weiterhin vergleichbare oder bessere antibakterielle Aktivitäten besitzen.

Aufgrund der pH-Abhängigkeit der gewählten Modifikation könnte eine enterische Antibiotikaformulierung (Freisetzung im Darm) z.B. mittels Kapseln besonders vorteilhaft sein. Alternativ dazu, könnte auch eine intravenöse Applikation erfolgen.

### Beschreibung der Figuren

**Figur 1****:** Struktur und Abbau von Cip-Hemiaminal ("Hemi", Verbindung 1). Gezeigt sind die molekularen Strukturen des beispielhaften Fluorchinolon-Derivates (Verbindung 1) und dessen Zerfallsprodukte Cip-d-CP und ein abbaubarer Linker 2-Hydroxy-tetrahydrofuran. 2-Hydroxytetrahydrofuran wird in gamma-Butyrolacton und gamma-Hydroxybutyrat umgewandelt. Diese Moleküle sind als in der aquatischen Umwelt biologisch abbaubar bekannt.
**Figur 2****:** Antibiotische Aktivität von Verbindung 1 (hier als "Hemi" bezeichnet) und Cip-d-CP. Gezeigt sind die minimalen Hemmkonzentrationen "MHK" [µg/ml] von Hemi im Vergleich zu Cip-D-CP in verschiedenen Bakterien (*E. faecalis* ATCC 29121, *P. aeruginosa* ATCC 27853, S. *aureus* ATCC 29213, *E*. *coli* ATCC 25922).
**Figur 3****:** Antibiotische Aktivität von Verbindung 1 (hier als "Hemi" bezeichnet) und Cip-d-CP. Gezeigt sind die minimalen Hemmkonzentrationen "MHK" [µg/ml] in verschiedenen Bakterienstämmen und Zytotoxizität in humanen HEK 293 Zellen von Hemi im Vergleich zu Cip-D-CP in verschiedenen Bakterien (*S*. *aureus* ATCC 43300, *E*. *coli* ATCC 25922, *K. pneumoniae* ATCC 700603, *A. baumanii* ATCC 19606, *P. aeruginosa* ATCC 27853 und humane embryonale Nierenzellen (HEK 293 CRL-1573)).
**Figur 4****:** Wachstumshemmung von *E*. *coli* durch Verbindung 1 ("Hemi") und Cip-d-CP. Gezeigt ist die Dosis-Wirkungsbeziehung der Wachstumshemmung von *E*. *coli* ATCC 23716 durch Verbindung 1 (hier als "Hemi" bezeichnet) im Vergleich zu Cip-d-CP
**Figur 5****:** Leuchthemmung in *V. fischeri* durch Verbindung 1 ("Hemi") und Cip-d-CP. Gezeigt ist die Dosis-Wirkungsbeziehung der Leuchthemmung der Bakterien *V. fischeri* durch Hemi im Vergleich zu Cip-d-CP.

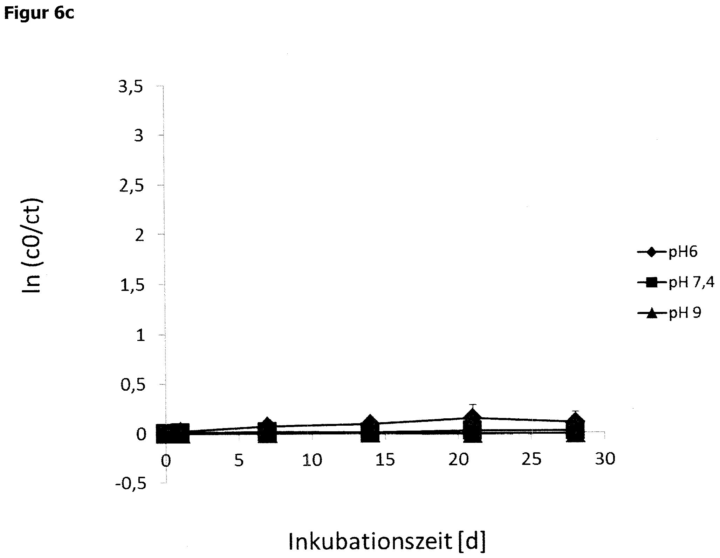

**Figuren 6a****-c:** Abiotische Hydrolyse von Verbindung 1 ("Hemi"), Cip-d-CP, und Ciprofloxacin. Gezeigt ist die pH-Abhängigkeit der abiotischen Hydrolyse von Hemi (Figur 6a) im Vergleich zu Cip-d-CP (Figur 6b) und Ciprofloxacin (Figur 6c). 10 mg/L der Testsubstanzen in Phosphat gepufferter Salzlösung wurden bei unterschiedlichen pH-Bedingungen bei 37°C (pH 6 und pH 7,4) oder bei Raumtemperatur (pH 9) für 28 Tage inkubiert und durch chromatographische Methoden analysiert. Die Ausgangskonzentration cₒ wurde mit der Konzentration am jeweiligen Zeitpunkt (ct) in Beziehung gesetzt und als natürlicher Logarithmus (in (c_{o/}ct)) gegen die Inkubationszeit aufgetragen.

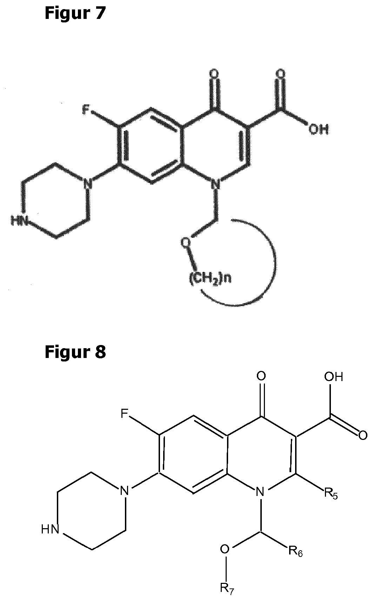

**Figur 7****:** Zyklische Hemiaminale.
**Figur 8****:** Offene Hemiaminale (nicht beansprucht).

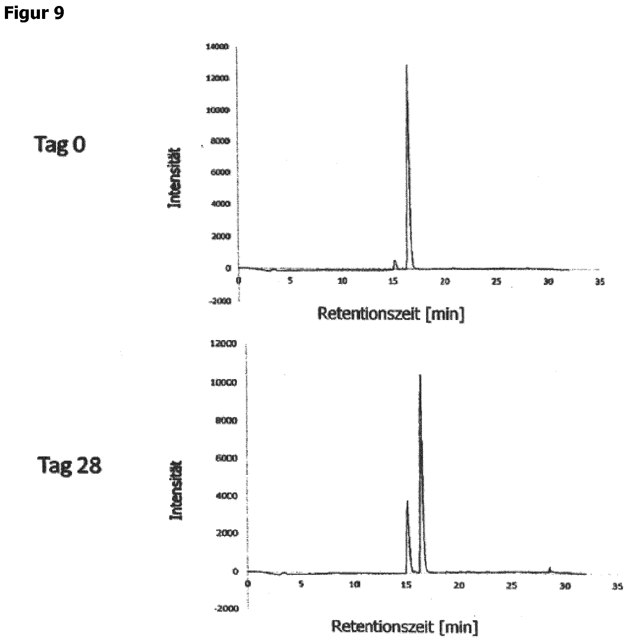

**Figur 9****:** Verbindung 1 ("Hemi") im Abbautest (geschlossener Flaschentest, OECD 301D),

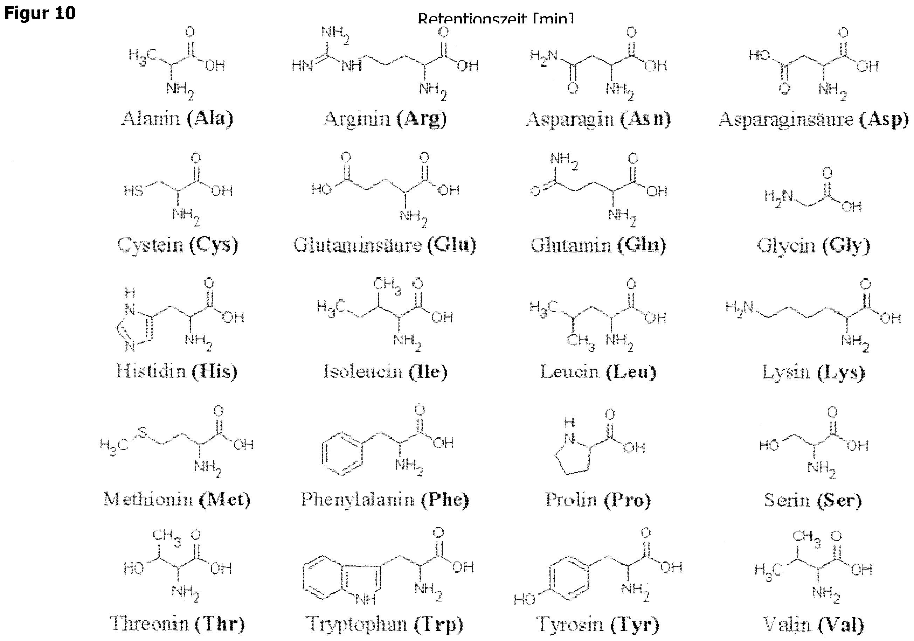

**Figur 10****:** Natürliche Aminosäuren.

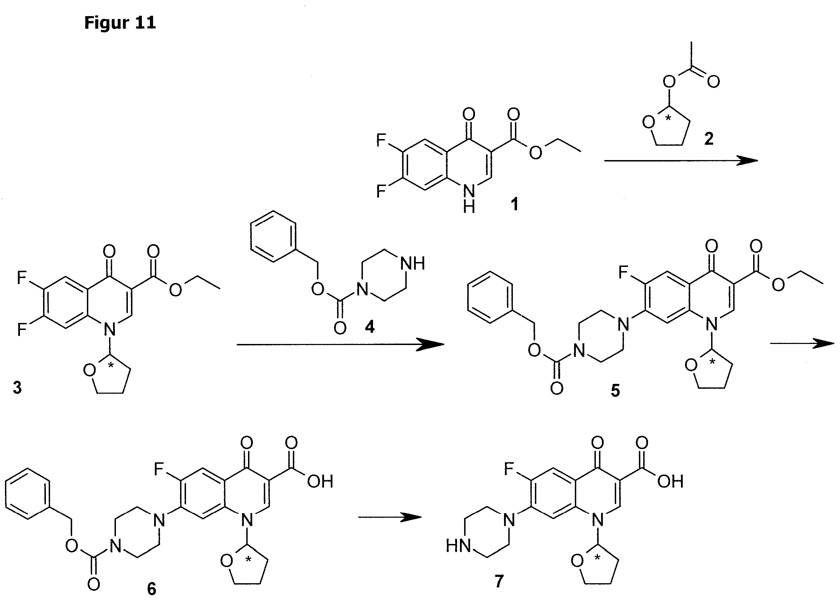

**Figur 11****:** Mögliches Syntheseschema für CIP-Hemi (siehe Verbindung 1 in Figur 1).

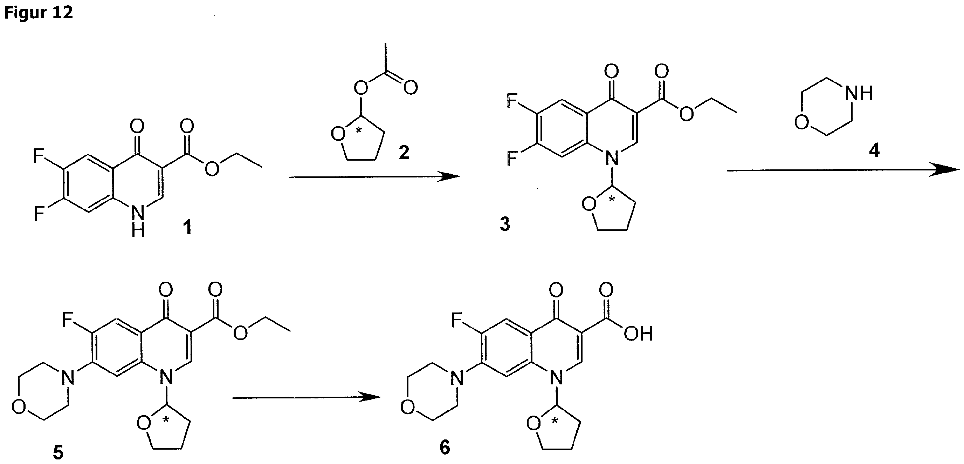

**Figur 12****:** Mögliches Syntheseschema für CG008-Hemi (nicht beansprucht) (siehe Verbindung 1 in Figur 1).

### Detaillierte Beschreibung der Erfindung

Die vorliegende Erfindung betrifft die folgenden Ausführungsformen:
Chinolon-Antibiotikum der allgemeinen Formel I: 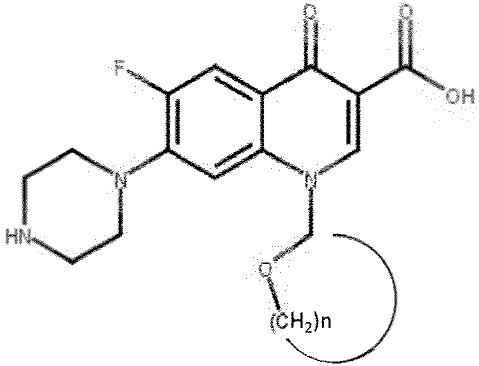 wobei n = 2-5.

Der +I-Effekt und der +M-Effekt der einzelnen Substituenten ist aus der chemischen Literatur wohl bekannt, er kann auch durch Berechnung der Elektronendichte am N1 modelliert werden.

Der Begriff "+I-Effekt" bezieht sich bevorzugt auf Alkyl-, Alkenyl-, Alkinyl-, Cycloalkyl-, Cycloaryl- und Heterocyclyl-Reste mit Substituenten, ausgewählt aus der Gruppe bestehend aus O-, -NH2, -NR2, -OH, -OR, -NH(CO)R, -O(CO)R, -(Aryl), Br, -Cl, -I, und -F, wobei R = C1-10 Alkyl.

Der Begriff "+I-Effekt" bezieht sich beispielsweise auf Cycloaryl-Reste mit +M (mesomerer Effekt)-Substituenten, wobei die +M-Substituenten bevorzugt ausgewählt sind aus der Gruppe bestehend aus, -O-, -NH2, -NR2, -OH, -OR, - NH(CO)R, -O(CO)R, -(Aryl), Br, -Cl, -I, und -F, wobei R = C1-10 Alkyl.

Soweit nicht anders angegeben, zielt die vorliegenden Erfindung auf alle Stereoisomere ab.

Die Erfindung betrifft auch ein Medikament enthaltend ein Chinolon-Antibiotikum nach Anspruch 1.

Die Erfindung betrifft auch ein Chinolon-Antibiotikum nach Anspruch 1 zur Verwendung bei der Behandlung von bakteriellen Infektionen.

Insbesondere betrifft die Erfindung die hierin beschriebenen Antibiotika zur Verwendung gegen Krankenhauskeime (nosokomiale Keime), bzw. Keime, die behandelt werden sollen, ohne eine Resistenzbildung bei anderen Keimen zu riskieren. Insbesondere betrifft die Erfindung die hierin beschriebenen Antibiotika zur Behandlung von *E*. *Coli*-verursachten Infektionen.

### Beispiele

### 1. Materialien

Verbindung 1 und andere erfindungsgemäße Antibiotika können auch unter Anwendung allgemeinen Fachwissens hergestellt werden.

### Wachstumshemmung in E. coli

*Escherichia coli K12 ATCC 23716* wurde von DSMZ GmbH (Braunschweig, Germany) erhalten.

Die Testsubstanzen wurden in Wasser verdünnt und auf einer 96-well-Platte zur Bakteriensuspension (in 2x Bakterienmedium (10 g Pepton and 6 g Fleischextrakt in 1 Liter)) gegeben, so dass die Endkonzentration des Mediums 1x war. Die Platte wurde 4h bei 37°C inkubiert. Die Zelldichte wurde photometrisch durch Messung der Absorption bei 600 nm bestimmt. Die Wachstumshemmung in Prozent der unbehandelten Kontrollen gegen die Konzentration der Testsubstanz aufgetragen.

### Zytotoxizitätstest

Humane embryonale Nierenzellen (HEK293) wurden in 384-well-Platten mit einer Dichte von 6000 Zellen/well in einem finalen Volumen von 50 µl mit der gewünschten Konzentration der Testsubstanz ausgesät. DMEM mit 10% fötalem Rinderserum (FBS) wurde als Wachstumsmedium verwendet. Die Zellen wurden zusammen mit den Testsubstanzen für 20 h bei 37 °C mit 5% COz inkubiert.

### Abiotische Hydrolyse:

Ciprofloxacin, Verbindung 1 und Cip-D-CP wurden in 12 ml phosphat-gepufferter Salzlösung in einer Konzentration von 10 mg/L gelöst. Der pH-Wert wurde auf pH 6, pH 7,4 oder pH 9 eingestellt. Die Ansätze wurden bei 37 °C für pH 6 und 7,4 und bei Raumtemperatur für pH 9 unter Schütteln (250 rpm) für 28 Tage inkubiert. An Tag 0, 1, 7, 14, 21 und 28 wurden Proben (700µL) für die HPLC Analyse entnommen. Der Quotient der Ausgangskonzentration cₒ durch die Konzentration am jeweiligen Zeitpunkt (cₜ) wurde kalkuliert und als natürlicher Logarithmus gegen die Inkubationszeit aufgetragen.

Minimale Hemmkonzentrationen in verschiedenen Bakterienstämmen:
Die antibiotische Aktivität wurde mittels Durchführung der Broth-Mikroverdünnungsmethode ermittelt (Cockerill et al., 2012, siehe Literatur). Es wurden die folgenden Bakterienstämme verwendet: *Staphylococcus aureus* (ATCC 29213), *Enterococcus faecalis* (ATCC 29212), *Escherichia coli* (ATCC 25922) und *Pseudomonas aeruginosa* (ATCC 27853), sowie *S. aureus* (ATCC 43300), *K. pneumoniae* (ATCC 700603), *A. baumanii* (ATCC 19606). Die Bestimmungen der minimalen Hemmkonzentrationen wurden durch zwei unabhängige Laboratorien durchgeführt.

Bakterienzellen wurden bei einer Zelldichte von 2 - 8 × 10⁵ cfu/ml in 200 µl Mueller-Hinton-Brothmedium verdünnt. Die Ansätze enthielten eine Verdünnungsreihe der Testsubstanzen. Diese inokulierten 96-well Platten wurden für 18 h bei 36°C inkubiert. Die minimale Hemmkonzentration wurde ermittelt durch visuelle Abschätzung der niedrigsten Konzentration, die die Bakterien vollständig gehemmt hatte.

### Leuchthemmung in V. fischeri

Die antibiotische Aktivität in *V. fischeri* wurden in einem Leuchtbakterientest (LBT) mit *Vibrio fischeri* NRRL-B-11177 (Hach-Lange GmbH, Düsseldorf, Germany) untersucht. Die Durchführung des LBT erfolgte nach dem modifizierten Protokoll von Menz et al. (2013), siehe Literaturliste.

Hochleistungschromatographie (HPLC), insbesondere für Ciprofloxacin, Verbindung 1 und Cip-d-CP:
Das Shimadzu Prominence HPLC System (Duisburg, Germany) wurde für die chromatographischen Untersuchungen der Testsubstanzen verwendet. Eine NUCLEODUR^{®} RP-C18 (CC 125/4 100-5µm C18 ec) Säule und mobile Phasen bestehend aus 0.1 % Ameisensäure in hochreinem Wasser (CH₂O₂: Solution A) and 100 % Acetonitril (CH₃CN: Solution B) wurden verwendet. Die Flußrate wurde auf 0.5 mL min⁻¹ eingestellt. Die Temperatur des Säulenofens wurde auf 25 °C eingestellt und das Injektionsvolumen betrug 10 µL. Ciprofloxacin, Hemi and Cip-D-CP eluierten bei den Retentionszeiten [t_{R}] 16,1 min, 16,5 min beziehungsweise 15,2 min. Die Testsubstanzen wurden durch einen UV/Vis Detektor bei 270 nm detektiert. Die Fließgradientenmethode wurde angewendet wie in Tabelle 1 aufgelistet.

**Tabelle 1 : Fließgradientenbedingungen zur chromatographischen Analyse der Testsubstanzen**

| **Zeit (min)** | **% Solution B (Acetonitril)** |
|---|---|
| 0.01-2.0 | 1 |
| 5.0 | 5 |
| 7.0 | 10 |
| 10.0 | 15 |
| 13.0 | 25 |
| 15.0 | 30 |
| 17.0 | 35 |
| 20.0 | 40 |
| 22.0 | 50 |
| 25.0 | 60 |
| 27.0 | 65 |
| 29.0 | 20 |
| 30.0-32.0 | 1 |
| 32.01 | Stop |

### 2. Synthese

### 2.1 CIP-Hemi

Ethyl-6,7-difluoro-4-oxo-1,4-dihydroquinolin-3-carboxylat (1) reagiert mit Tetrahydrofuran-2-ylacetat (2) bei ≥120°C zu Ethyl-6,7-difluoro-4-oxo-1-(tetrahydrofuran-2-yl)-1,4-dihydroquinolin-3-carboxylat (3) (siehe Yamashita et al. 1983). Nachfolgend wird (3) mit Benzylpiperazine-1-carboxylat (4) durch eine SN Reaktion zu Ethyl-7-(4-«benzyloxy)carbonyl)piperazin-1-yl)-6-fluoro-4-oxo-1-(tetrahydrofuran-2-yl)-1,4-dihydroquinoline-3-carboxylat (5) umgesetzt. Als Lösungsmittel können DMSO oder Acetonitril (ACN) mit Triethylamin (TEA) verwendet werden. Anschließend wird (5) mit NaOH zu 7-(4-((benzyloxy)carbonyl)piperazin-1-yl)-6-fluoro-4-oxo-1-(tetrahydrofuran-2-yl)-1,4-dihydroquinoline-3-carbonsäure (6) hydrolysiert und die Cbz(Benzyloxycarbonyl)-Gruppe mit z.B. Pd/C H₂ entfernt (7).

### Anmerkungen:

▪ Ethyl 6,7-difluoro-4-oxo-1,4-dihydroquinolin-3-carboxylate (1), Cas. 121873-01-6, von ABCR, AB375454
▪ 1-Carbobenzoxypiperazine (4), Cas. 31166-44-6, von Chempur, BD28331-5
▪ Tetrahydro-2-furanyl acetate (2), Cas. 1608-67-9, kann nach Yamashita et al. 1983 synthetisiert werden

### 2.2 CG008-Hemi (nicht beansprucht)

Die Synthese von CG008-Hemi erfolgt analog zu CIP-Hemi. Ethyl-6,7-difluoro-4-oxo-1,4-dihydroquinolin-3-carboxylate (1) reagiert mit Tetrahydrofuran-2-ylacetat (2) bei ≥120°C zu Ethyl-6,7-difluoro-4-oxo-1-(tetrahydrofuran-2-yl)-1,4-dihydroquinolin-3-carboxylat (3) (siehe Yamashita et al. 1983). Nachfolgend reagiert dieses mit Morpholin (4) in z.B. ACN mit TEA zu Ethyl-6-fluoro-7-morpholino-4-oxo-1-(tetrahydrofuran-2-yl)-1,4-dihydroquinolin-3-carboxylat (5). Abschließend wird der Ester mit NaOH zu CG008-Hemi (6) hydrolysiert.

### Anmerkungen:

▪ Ethyl 6,7-difluoro-4-oxo-1,4-dihydroquinolin-3-carboxylat, Cas. 121873-01-6, von ABCR, AB375454
▪ Tetrahydro-2-furanylacetat, Cas. 1608-67-9, kann nach Yamashita et al. 1983 synthetisiert werden
▪ Morpholin, Cas. 110-91-8, von Carl Roth, 9691.1

### 2.3 Allgemeine Anmerkungen

Die vorgestellten Synthesen stellen Standardmethoden in der Synthese von Fluoroquinolonen dar (Gould-Jacobs-Reaktion, SN am Aromaten und Hydrolyse des Esters). Weiterhin ist auch das Schützen mit einer Cbz-Gruppe eine Standardmethode. Für die Synthese des Hemi-Linkers können wiederum verschiedene Möglichkeiten genutzt werden, welche aus der Synthese des Zytostatikums Tegafur bekannt sind - siehe auch Lukevits & Zablotskaya 1991. Weitere generelle Synthesemöglichkeiten für Antibiotika bzw. chemische Reaktionen zum Einfügen bestimmter Gruppen eines Moleküls werden auch in der hierin zitierten Literatur beschrieben.

### Zitierte Literatur:

EP0373531
Takenouchi, T, Tabata, F., Iwata, Y., Hanzawa, H. Sugawara, M. and Ohya, S. (Hydrophilicity of quinolones is not an exclusive factor for decreased activity in efflux-mediated resistant mutants of Staphylococcus aureus), Antimicrobial Agents and Chemotherapy, 1996, 40(8), 1835-1842
Cockerill, Franklin R. (2012): Methods for dilution antimicrobial susceptibility tests for bacteria that grow aerobically. Approved standard - ninth edition. Wayne, Pa.: CLSI (Clinical and Laboratory Standards Institute, M07-A9 = 32,2).
Menz, J.; Schneider, M.; Kümmerer, K. (2013): Toxicity testing with luminescent bacteriacharacterization of an automated method for the combined assessment of acute and chronic effects. In: Chemosphere 93 (6), S. 990-996. DOI: 10.1016/j.chemosphere.2013.05.067.
Radi et al.; Collection of Czechoslovak Chemical Communications, 1992, 57(1), 216-18.
Yamashita et al., "Studies on Tetrahydrofuryl-5-fluorouracils. IV. Mode of Reaction of 5-Fluorouracil with 2-Acetoxytetrahydrofuran", 1983 Chem. Pharm. Bull., 31, Seiten 3872-3877.
Lukevits & Zablotskaya 1991, "Synthesis of Ftorafur", Chemistry of Heterocyclic Compounds c/c of Khimiia Geterotsiklicheskikh Soedinenii; 27, 12; 1271.
WO2015/189560
DE 692 32 134
WO 01/36408 A1
DE 36 32 222 A1

## Patentansprüche

1. Chinolon-Antibiotikum der allgemeinen Formel I: 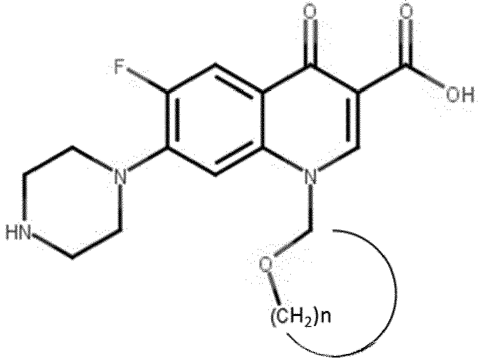 wobei n = 2-5.

2. Medikament enthaltend ein Chinolon-Antibiotikum nach Anspruch 1.

3. Chinolon-Antibiotikum nach Anspruch 1 zur Verwendung bei der Behandlung von bakteriellen Infektionen.

## Claims

1. Quinolone antibiotic of the general formula I: 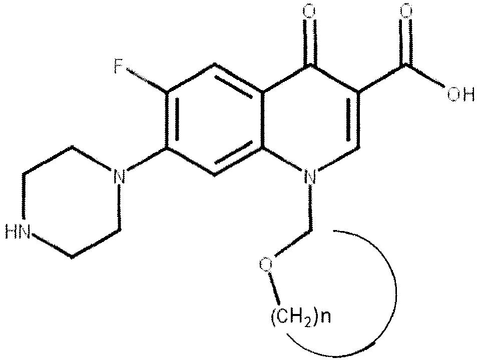 wherein n = 2-5.

2. Medicament containing a quinolone antibiotic according to claim 1.

3. Quinolone antibiotic according to claim 1 for use in the treatment of bacterial infections.

## Revendications

1. Antibiotique de type quinolone de formule générale I : 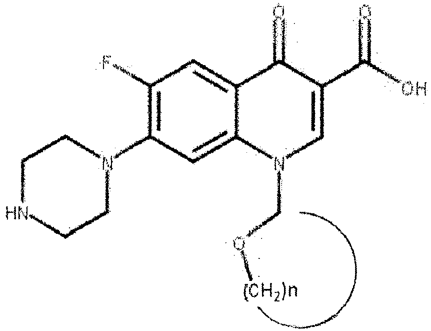 dans laquelle n = 2 à 5.

2. Médicament contenant un antibiotique de type quinolone selon la revendication 1.

3. Antibiotique de type quinolone selon la revendication 1, destiné à être utilisé dans le traitement d'infections bactériennes.
